# EUROPÄISCHE PATENTANMELDUNG

(11) **EP 1 344 506 A1**
(43) Veröffentlichungstag der Anmeldung: **17.09.2003**
(21) Anmeldenummer: 02005630.5
(22) Anmeldetag: 12.03.2002
(51) Int. Cl.: A61F 2/44

(54) **Zwischenwirbelprothese für die Halswirbelsäule**

(71) Anmelder: Waldemar Link (GmbH & Co.), 22339 Hamburg (DE)
(72) Erfinder: Keller, Arnold, 23863 Kayhude (DE); McAfee, Paul C.,M.D.c/o Scoliosis and Spine Center, Baltimore, MD 21204 (US)
(74) Vertreter: Glawe, Delfs, Moll & Partner

(57) **Zusammenfassung**

Zwischenwirbelprothese für die Halswirbelsäule mit zwei jeweils mit einem Wirbelkörper zu verbindenden Deckplatten (10, 11), die einen ventralen Befestigungsflansch (25, 26) mit Stiftlöchern (27) für Befestigungsstifte (5) aufweisen. Die Stiftlöcher (27) sind als richtungsbestimmende Führungslöcher mit unparalleler Richtung ausgebildet. Es ist ihnen eine Sicherungseinrichtung (28, 37) zugeordnet, die das Austreten der Befestigungsstifte verhindert.

## Beschreibung

Es sind Zwischenwirbelprothesen zum Ersatz der Bandscheibe bekannt, die aus zwei gelenkig miteinander verbundenen Deckplatten bestehen, die an ihrem ventralen Rand mit einem lotrecht zu ihrer sonstigen Erstreckung verlaufenden Sicherungsflansch verbunden sind, der sich an die ventrale Fläche des zugehörigen Wirbelkörpers anlegt und mit diesem verschraubt wird (US-A-5,258,031). Im Bereich der Lendenwirbelsäule sind die Wirbelkörper so groß, daß kräftige Knochenschrauben verwendet werden können, deren langzeitige Verankerung im Knochen von vielen Fachleuten als hinreichend sicher angesehen wird. Auch kann man im Lendenwirbelbereich mehrere Sicherungsflansche nicht nur an der ventralen, sondern auch an lateralen Seiten des Wirbelkörpers anbringen (US-A-4,759,769), um dadurch eine besonders sichere Verankerung der Prothese am Wirbelkörper zu erreichen. Diese Möglichkeiten bestehen im Bereich der Halswirbelsäule nicht. Die Wirbelkörper sind so klein und flach, daß die Befestigungsschrauben kaum dicker als 2 mm sein können und dann ein so feines Gewinde tragen, daß dieses die Schrauben nicht mit Sicherheit gegenüber in ihrer Längsrichtung wirkenden Kräften verankern kann. Auch kann der Sicherungsflansch nur ventral vom Wirbelkörper vorgesehen werden, weil eine seitliche Anordnung durch die dort befindlichen Wirbelfortsätze ausgeschlossen wird.

Die Erfindung sucht nach einer sicheren Verankerung der Prothese an den Wirbelkörpern der Halswirbelsäule und erreicht diese durch die Merkmale des Anspruchs 1 und vorzugsweise diejenigen der Unteransprüche. Danach ist vorgesehen, daß der Befestigungsflansch Stiftlöcher für Befestigungsstifte aufweist, die als richtungsbestimmende, mit unparalleler Richtung angeordnete Führungslöcher für die Befestigungsstifte ausgebildet sind. Die Führungslöcher halten die Befestigungsstifte in unparalleler Richtung, so daß eine Lösung des Befestigungsflansches von der ventralen Wirbelkörperseite nur denkbar ist unter gleichzeitiger Querverlagerung der Stifte innerhalb des Wirbelkörpers, was äußerst unwahrscheinlich ist. Dadurch wird ein hoher Sicherheitsgrad für die Befestigung der Prothese an den Wirbelkörpern erreicht.

Damit die Stifte sich nicht in Längsrichtung aus der Sicherungsstellung entfernen können und gegebenenfalls ihre hervortretenden Enden benachbarte Organe gefährden, ist zweckmäβigerweise eine Sicherungseinrichtung zum Verhindern des Austretens der Befestigungsstifte aus den Stiftlöchern vorgesehen. Diese Sicherungseinrichtung ist eine Sicherungsplatte, die an dem Befestigungsflansch beweglich ist zwischen einer Freistellung, in der sie die Stiftlöcher zur Montage frei gibt, und einer Sicherungsstellung, in der sie das Stiftende der in den Stiftlöchern befindlichen Stifte mindestens teilweise überdeckt, so daß sie nicht austreten können. Die Sicherungsplatte ist vorteilhafterweise eine unter Vorspannung stehende Federplatte. Deren Vorspannung dient nicht nur dazu, die Stifte um so wirksamer zurückzuhalten, sondern auch dazu, die Platte in ihrer jeweiligen Stellung aufgrund der durch die Reibung verursachten Vorspannung und gegebenenfalls auch eines teilweisen Formschlusses in ihrer Sicherungsstellung festzuhalten.

Besonders zweckmäßig ist eine Ausführungsform, bei der zwei Stiftlöcher an dem Befestigungsflansch vorgesehen sind und die Sicherungsplatte zwischen diesen drehbar angebracht ist, um auf beide zu wirken.

Wenn die Prothese einen Prothesenkern aufweist, der in einem Einschubsitz der Deckplatte gehalten ist, kann die Sicherungs- oder Federplatte auch dazu dienen, den Prothesenkern in seinem Einschubsitz zu halten, indem sie einen Anschlag bildet, der in der Sicherungsstellung im Einschubweg des Prothesenkerns liegt.

Die Erfindung wird im folgenden unter Bezugnahme auf die Zeichnung näher erläutert, die ein vorteilhaftes Ausführungsbeispiel veranschaulicht. Es zeigen:
- Fig. 1: eine Draufsicht auf einen Halswirbel,
- Fig. 2: eine vergrößerte, schematische Darstellung des Wirbelkörpers mit einem Schnitt durch einen Befestigungsflansch,
- Fig. 3: die Zerlegungsansicht einer praktischen Ausführungsform der Prothese und
- Fig. 4: die untere Deckplatte und den Prothesenkern der Ausführungsform gemäß Fig. 3 im montierten Zustand.

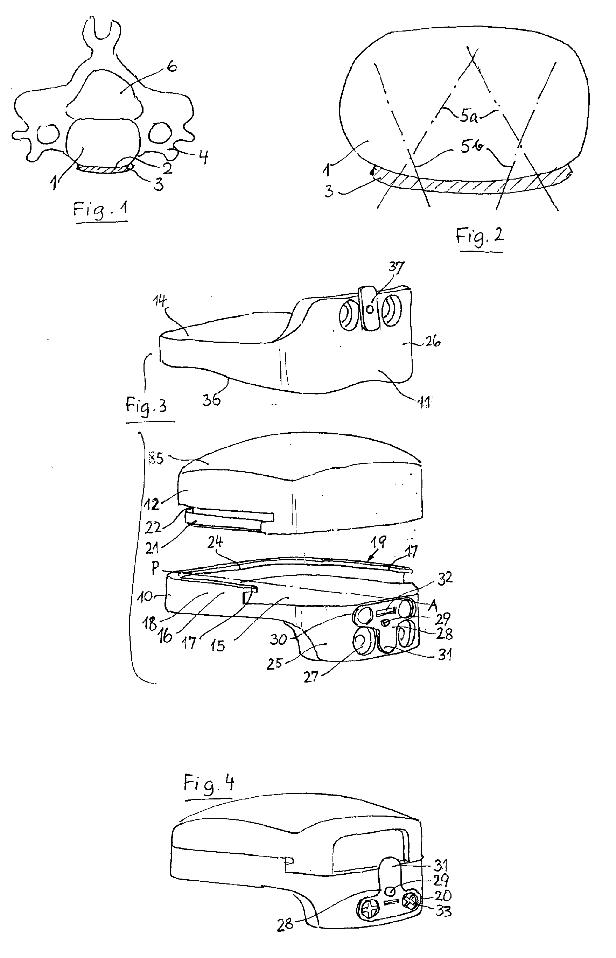

Anhand von Fig. 1 erkennt man, daß am Wirbelkörper 1 lediglich die ventrale Stirnseite 2 zur Anbringung des Befestigungsflansches 3 einer Zwischenwirbelprothese in Betracht kommt, weil die lateralen Flächen durch Wirbelfortsätze 4 eingenommen sind. Man erkennt ferner, daß der Wirbelkörper sehr klein ist und bei einer Höhe von wenigen Millimetern die Befestigungsmöglichkeiten stark eingeschränkt sind.

Fig. 2 veranschaulicht das erfindungsgemäße Befestigungsprinzip. An dem Wirbelkörper 1 wird der Befestigungsflansch 3 mittels eines Paares von Stiften 5a bzw. 5b befestigt, deren Richtung durch die für sie vorgesehenen Stiftlöcher in dem Befestigungsflansch 3 unparallel vorgegeben ist. Sie schließen miteinander einen Winkel ein, der vorzugsweise größer ist als 15°, weiter vorzugsweise größer als 20°. Bei den Stiften kann es sich um feine Knochenschrauben handeln. Die Stifte 5a dringen konvergierend und die Stifte 5b divergierend in den Wirbelkörper ein.

Normalerweise werden lediglich zwei derartiger Stifte verwendet. Die Erfindung schließt aber die Verwendung einer größeren Zahl von Stiften nicht aus.

Fig. 3 und 4 veranschaulichen die praktische Ausführung der Erfindung. Die Prothese besteht aus einer unteren Deckplatte 10, einer oberen Deckplatte 11 und einem Prothesenkern 12, deren Umriß in der Ansicht von oben etwa demjenigen des Wirbelkörpers 1 gleicht. Die Deckplatten 10, 11 weisen Außenflächen 14 auf, die zur Verankerung an zugehörigen Wirbelkörpern bestimmt sind. Sie sind vorzugsweise eben. Es sind aber auch andere, im wesentlichen flache Gestaltungen einschließlich geeigneter Oberflächenstrukturen zur besseren Verankerung am Knochen denkbar. Sie bestehen vorzugsweise aus Metall. Sie sind in der Draufsicht etwa rechteckig mit abgerundeten Ecken oder oval mit größerer Abmessung in Querrichtung als in AP-Richtung. Die AP-Richtung ist in Fig. 3 durch die strichpunktierte Linie A-P angedeutet.

Die untere Deckplatte 10 wendet der oberen Deckplatte 11 eine ebene Oberseite 15 zu, die auf drei Seiten von einem Kragen 16 eingefaßt ist, der oberhalb eines Hinterschnitts eine nach innen ragende Leiste 17 bildet. Die seitlichen Abschnitte 18, 19 des Kragens 16 verlaufen zueinander parallel und geradlinig.

Die Oberfläche 15 und der Kragen 16 der unteren Deckplatte 10 bilden einen Sitz für den Prothesenkern 12. Dieser besteht aus gleitgünstigem Werkstoff, beispielsweise Polyethylen. Er hat eine zur Fläche 15 passende ebene Unterfläche, die zumindest an den Seiten von einer Randleiste 21 begrenzt wird, oberhalb welcher sich eine Nut 22 befindet. Die Leiste 21 greift im montierten Zustand in den Hinterschnitt des Kragens 16 unterhalb der Leiste 17 ein. Die Leiste 17 greift in die Nut 22 ein.

Die seitlichen Abschnitte 18, 19 des Kragens 16 bilden eine Führung für den Prothesenkern 12, in welcher dieser von der offenen, ventralen Seite (in Fig. 3 rechts vorne) her eingeschoben werden kann. Die seitlichen Abschnitte 18, 19 des Kragens 16 sind auf der dorsalen Seite (in Fig. 3 links hinten) durch einen Abschnitt 24 des Kragens 16 verbunden, der dieselbe Querschnittsgestalt wie die seitlichen Abschnitte 18, 19 haben kann; erforderlich ist dies jedoch nicht. Der dorsale Abschnitt 24 des Kragens 16 wirkt als Sicherungsanschlag, der ein Entweichen des Kerns 12 aus dem Zwischenraum zwischen den Deckplatten 10, 11 zum Rückenmarkskanal 6 hin verhindert. Es versteht sich, daß die gegenseitige Führung des Sitzes 15, 16 und des Prothesenkerns 12 auch in anderer Weise ausgeführt sein kann.

An ihrem ventralen Rand weisen die Deckplatten 10, 11 je einen rechtwinklig von ihnen abragenden Befestigungsflansch 25, 26 auf, der Schraubenlöcher 27 zur Befestigung am Wirbelkörper 1 enthält. Diese sind als Führungslöcher für die Schrauben ausgebildet. Das heißt, daß sie die Richtung der Schrauben innerhalb eines eng begrenzten Winkelbereichs vorgeben. Die Führungslöcher und die Schrauben sollen so ausgeführt sein, daß die maximale Winkelabweichung der Schrauben von der Richtung der Führungslöcher zu jeder Seite hin nicht größer als 7°, vorzugsweise nicht größer als 4°, ist.

Am Flansch 25 der unteren Deckplatte 10 ist mittig zwischen zwei Schraubenlöchern 27 und etwas höher als diese eine Sicherungsplatte 28 mittels eines Kopfbolzens 29 drehbar befestigt. Sie weist zwei sich im wesentlichen zur Seite hin erstreckende Flügel 30 und eine sich quer dazu erstreckende Zunge 31 auf. Sie besteht aus federndem Metall und ist so vorgespannt, daß ihre Flügel 30 gegen die untere Deckplatte 10 drücken. Zum Eingriff eines Drehwerkzeugs, beispielsweise eines Schraubenziehers, enthält sie eine passende Öffnung oder Vertiefung 32. Wenn sie sich in der in Fig. 3 dargestellten, montagebereiten Stellung befindet, ragt sie nicht über die Oberfläche 15 der unteren Deckplatte 10 hinaus. Der Prothesenkern 12 kann daher unbehindert in die von den Kragen 16 gebildete Einschubführung eingeschoben werden. Wird sie um 180° gedreht, wie es in Fig. 4 dargestellt ist, ragt die Zunge 31 über die Oberfläche 15 und bildet dadurch einen Anschlag, der den Kern 12 am Verlassen der Führung zur ventralen Seite hin hindert.

In der in Fig. 3 gezeigten Montagestellung läßt die Sicherungsplatte 28 die Schraubenlöcher 27 für die Montage der Knochenschrauben frei. Wie in Fig. 4 gezeigt, überdecken die Flügel 30 in der Sicherungsstellung ganz oder teilweise die Schraubenlöcher 27 und drücken elastisch auf die darin befindlichen Schraubenköpfe, um sie am Heraustreten aus den Schraubenlöchern 27 zu hindern. Die Sicherungsplatte wird in der Sicherungsstellung durch die Reibung gehalten, die auf ihrer federnden Vorspannung beruht. Zusätzlich kann die Form ihrer Flügel 30 derjenigen der Schraubenköpfe derart angepaßt sein, daß die Sicherungsplatte durch Formschluß gehindert ist, sich aus der Sicherungsstellung herauszudrehen, beispielsweise durch Löcher 33, die unter der Vorspannung der Platte über den Kuppen der Schraubenköpfe einrasten.

Eine entsprechende Sicherungseinrichtung ist für die Schraubenlöcher 27 im Befestigungsflansch 26 der oberen Deckplatte 11 vorgesehen. Diese Sicherungseinrichtung kann ebenfalls als drehbare Federplatte 37 ausgeführt sein, deren Flügel sich im montierten Zustand über die Schraubenlöcher 27 bzw. über die darin befindlichen Schraubenköpfe legen. Es versteht sich, daß diese Sicherungsplatte keine Anschlagzunge 31 zu haben braucht.

Oberseitig weist der Kern 12 eine vorzugsweise konvexsphärische Gelenkfläche 36 auf, die zur Bildung eines Gelenks zusammenwirkt mit der unterseitigen, konkav-sphärischen Gleitfläche 26 der oberen Deckplatte 11. Der Prothesenkern 12 hat allseits im wesentlichen dieselbe Umrißgestalt in der Draufsicht wie die untere und obere Deckplatte 10, 11. Er überdeckt insbesondere den Kragen 16, so daß die Größe der von dem Kern zur Verfügung gestellten Gleitfläche 35 durch das Vorhandensein des Kragens 16 nicht verringert wird. Der Prothesenkern kann dem Zwischenraum zwischen den Deckplatten 10, 11 nicht entweichen, weil er durch das Zusammenspiel der hinterschnittenen Leisten 17 und 21 nicht nur an seitliche Bewegung, sondern auch am Abheben von der Deckplatte 10 gehindert ist.

## Patentansprüche

1. Zwischenwirbelprothese für die Halswirbelsäule mit zwei jeweils mit einem Wirbelkörper (1) zu verbindenden Deckplatten (10, 11), die einen ventralen Befestigungsflansch (25, 26) mit Stiftlöchern (27) für Befestigungsstifte (5) aufweisen, **dadurch gekennzeichnet, daß** die Stiftlöcher (27) als richtungsbestimmende, mit unparalleler Richtung angeordnete Führungslöcher für die Befestigungsstifte (5) ausgebildet sind.

2. Prothese nach Anspruch 1, **dadurch gekennzeichnet, daß** den Stiftlöchern (27) eine Sicherungseinrichtung (28, 37) zum Verhindern des Austretens der Befestigungsstifte (5) zugeordnet ist.

3. Prothese nach Anspruch 2, **dadurch gekennzeichnet, daß** die Sicherungseinrichtung eine Sicherungsplatte (28, 37) umfaßt, die an dem Befestigungsflansch (25, 26) beweglich ist zwischen einer Freistellung und einer Sicherungsstellung, in welcher sie das Stiftende überdeckt.

4. Prothese nach Anspruch 3, **dadurch gekennzeichnet, daß** die Sicherungsplatte (28, 37) eine unter Vorspannung stehende Federplatte ist.

5. Prothese nach Anspruch 3 oder 4, **dadurch gekennzeichnet, daß** die Sicherungsplatte (28, 37) zwischen zwei Stiftlöchern (27) an dem Befestigungsflansch (25, 26) drehbar angebracht ist.

6. Prothese nach einem der Ansprüche 2 bis 5, **dadurch gekennzeichnet, daß** sie einen Prothesenkern (12) aufweist, der in einem Einschubsitz (16, 17) der Deckplatte (10) gehalten ist, und daß die Sicherungseinrichtung (28) einen Teil (31) aufweist, der in der Sicherungsstellung als Anschlag im Einschubweg des Prothesenkerns (12) liegt.
